# EUROPEAN PATENT SPECIFICATION

(11) **EP 0 359 341 B1**
(45) Date of publication and mention of the grant of the patent: **16.02.1994**
(21) Application number: 89202341.7
(22) Date of filing: 15.09.1989
(51) Int. Cl.: C07D 487/10, C07D 519/00, C08G 59/06, C08G 59/26

(54) **1,6-diazaspiro[4,4]nonane-2,7-dione derivatives**
1,6-Diazaspiro[4,4]nonane-2,7-dionderivate
Dérivés de 1,6-diazaspiro[4,4]nonane-2,7-diones

(30) Priority: 16.09.1988 US 245433; 17.03.1989 US 324870; 16.09.1988 US 245618; 16.09.1988 US 245619; 16.09.1988 US 245434; 27.09.1988 US 249934
(43) Date of publication of application: 21.03.1990
(73) Proprietor: SHELL INTERNATIONALE RESEARCH MAATSCHAPPIJ B.V., 2596 HR Den Haag (NL)
(72) Inventor: Wang, Pen Chung, Houston Texas 77079 (US)

(56) References cited:
- None

## Description

The invention relates to the production of certain novel 1,6-diazaspiro[4,4]nonane-2,7-dione derivatives.

More particularly the invention relates to novel 1,6-diazaspiro[4,4]nonane-2,7-dione derivatives having hydroxyaryl containing substituents on the ring nitrogen atoms of the spirodilactam ring system, and
i) whereof the two adjacent ring atoms on the 3-, 4-, and/or 8-, 9-positions form part of a benzene ring and/or
ii) whereof the hydroxy groups of the hydroxyaryl containing substituent are reacted to produce unsaturated ester or ether derivatives.

Reaction products of epichlorohydrin and spirobiindanols are disclosed in U.S. 4,672,102 wherein the resulting polyhydric polyethers are said to have high heat distortion temperatures. The values reported are from about 131 °C to about 153 °C, depending upon the nature of the substituents present. The corresponding value for the polyhydric polyether of 2,2-di(4-hydroxyphenyl)propane was 88 °C. It would be of advantage to provide a class of novel starting materials being or leading to phenoxy-type resins having comparable or even higher glass transition temperatures. Unsaturated ether or ester derivatives of polyhydric phenols are well known as a class of compounds and much of the technology related herewith is common knowledge. Illustrative is US patent No. 4,100,140 which discloses the reaction of 2,2-di(4-hydroxyphenyl)propane, also known as bisphenol A or BPA, with allylchloride in the presence of sodium salt, to produce 2,2-di(4-allyloxyphenyl)propane which can be cured by for instance contacting the diallyl ether with an imide containing curing agent. U.S. patent No. 4,468,524 relates to cured acrylate- and methacrylate esters of polyhydric phenols. In particular it was found that cured products from unsaturated derivatives of aromatic phenolic compounds having polycyclic structures wherein some or all the rings share common atoms with other rings of the polycyclic structure provide desirable properties in high temperature applications. Therefore, it would be an advantage to provide a novel class of unsaturated derivatives of phenolic compounds having a plurality of rings within the molecular structure, which react with conventional curing agents to produce insoluble products having a comparable or even better high temperature performance.

Novel 1,6-diazaspiro[4,4]nonane-2,7-dione derivatives are disclosed in European Patent 0336475.

The novel 1,6-diazaspiro[4,4]nonane-2,7-dione derivatives of this invention are these having the formula I: 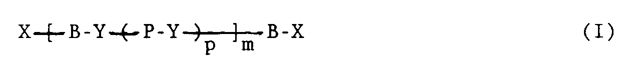
in which B has the formula II: 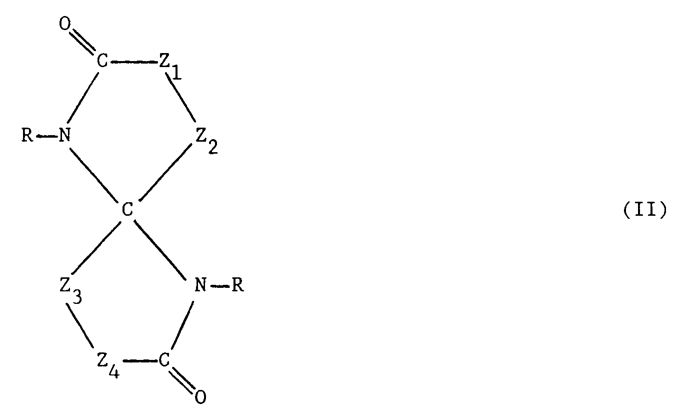
wherein
a) Z₁, Z₂, Z₃, Z₄ are independently
   - C(Z')₂ in which Z' independently is hydrogen or C₁₋₄ alkyl, or
   - such that two adjacent Z atoms form part of a benzene ring;
b) each R is independently a bivalent group of the formula III or IV: 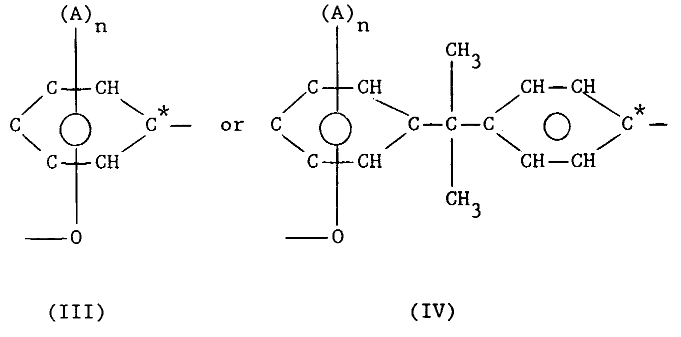 wherein A is a substituent replacing hydrogen selected independently from a C₁₋₄ alkyl or halogen atom, n independently is 0, 1 or 2 and the free valence bond of C* is linked with a ring nitrogen atom of the spirodilactam of the formula II;
c) m ≧ 0;
d) each X independently is hydrogen or a glycidyl-, acrylyl-, methacrylyl-, allyl- or propargyl group, with the proviso that when X is acrylyl, methacrylyl, allyl or propargyl then m is 0;
e) Y is 2-hydroxy-1,3-propadiyl;
f) each P independently is a di(oxyphenyl)propane moiety;
g) p is on average of from 0 to 3;
with the proviso that when R is a bivalent group of the formula III and simultaneously Z₂ and Z₃ are each CH₂ and simultaneously Z₁ and Z₄ are C(Z')₂ wherein each Z' independently represents hydrogen or a C₁₋₄ alkyl, then each X is not hydrogen or glycidyl.

The difunctional polycyclic hydroxy aryl containing 1,6-diazaspiro[4,4]nonane-2,7-diones of the invention in which m is 0, may also be referred to as dihydric spirodilactams. They can be prepared by reacting in liquid phase a solution of a hydroxy containing primary amino compound of the formula V:

H―R―NH₂ (V)

wherein R has the same meaning as defined before and wherein the amino group is bonded to the C* of R and the hydrogen is bonded to the oxygen radical of R respectively, with either a 4-oxoheptanedioic acid compound or an ester- or acid halide derivative thereof (reactant VIa) of the formula VI: 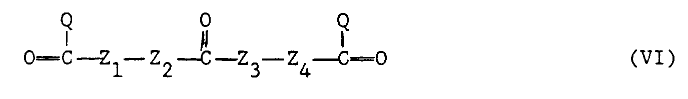
or a 1,6-dioxaspiro[4,4]nonane-2,7-dione (reactant VIb) of the formula VII: 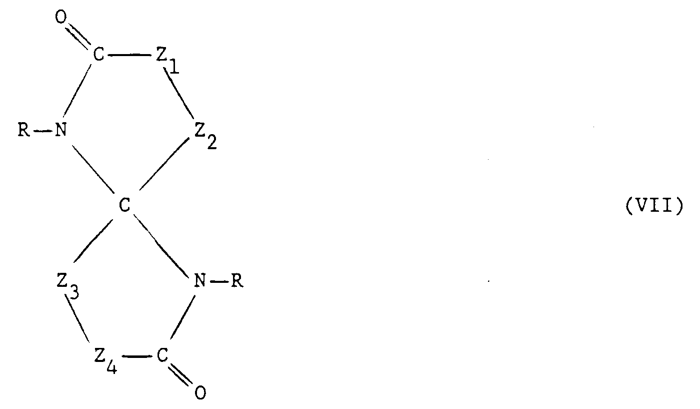
wherein R and Z₁, Z₂, Z₃ and Z₄ for both reactants VIa and VIb have the same meaning as defined before and Q is hydroxy, lower alkoxy of up to 4 carbon atoms or halide, preferably chloride or bromide.

Illustrative dihydric spirodilactams include
1,6-di(4-hydroxyphenyl)-3,3,4,4,8,8,9,9-octamethyl-1,6-diazaspiro[4,4]nonane-2,7-dione,
1,6-di[4-(4-hydroxyphenylisopropyl)phenyl]-1,6-diazaspiro[4,4]nonane-2,7-dione,
1,6-di(4-hydroxyphenyl)-3,4,8,9-dibenzo-1,6-diazaspiro[4,4]nonane-2,7-dione,
1,6-di(3-hydroxyphenyl)-3,4,8,9-dibenzo-1,6-diazaspiro[4,4]nonane-2,7-dione,
1,6-di(3-hydroxy-4-chlorophenyl)-3,4-dimethyl-1,6-diazaspiro[4,4]nonane-2,7-dione,
1,6-di(3-hydroxy-4-chlorophenyl)-3,4,8,9-tetramethyl-1,6-diazaspiro[4,4]nonane-2,7-dione,
1,6-di(3-hydroxy-4-methylphenyl)-3,3,4,4,8,8,9,9-octamethyl-1,6-diazaspiro[4,4]nonane-2,7-dione,
1,6-di(4-hydroxyphenyl)-3,4-benzo-8-methyl-1,6-diazaspiro[4,4]nonane-2,7-dione.

The glycidyloxy-substituted spirodilactams according to the present invention are produced from the dihydric spirodilactams by conventional technology typically employed for the production of glycidyloxyphenyl-containing epoxy compounds and the acrylyl-, methacrylyl-, allyl- or propargyloxy substituted spirodilactams according to the present invention are produced by conventional technology typically employed for the production of unsaturated esters or ethers of polyhydric phenols.

Preferably the glycidyl-, acrylyl-, methacrylyl-, allyl- or propargyloxy substituted spirodilactam compound is represented by the formula I wherein each Z independently is C(Z')₂ in which each Z' independently is hydrogen or methyl or wherein Z₁ and Z₂ and/or Z₃ and Z₄ atoms form part of a benzene ring. Particularly preferred are spirodilactams of the formula I wherein n is 0. Spirodilactams of the formula I wherein R is a compound of the formula III, wherein n is 0 and wherein each Z independently is C(Z')₂ in which each Z' independently is hydrogen or methyl or wherein Z₁ and Z₂ and/or Z₃ and Z₄ form part of a benzene structure fused to the spiro ring system being most preferred.

The X oxyaryl substituent may be a 3-, or a 4-X-oxyarylsubstituent, the latter being most preferred.

Illustrative hydroxy containing primary amino compounds include p-aminophenol, m-aminophenol and 2-(4-aminophenyl)-2-(4-hydroxyphenyl)propane, preferred primary amino compounds of the formula V are those wherein R is a compound of the formula III and wherein n is 0.

Many of the acyclic 4-oxoheptanedioic acid compounds or its derivatives according to the formula VI are known. Certain of the esters can also be produced by the reaction of formaldehyde and unsaturated carboxylic acid esters as disclosed in U.S. patent No. 4,800,231. Interconversion of the acids, esters or acid halides of compounds according to the formula VI can be done by conventional methods. The process for the production of 4-oxoheptanedioic acid compounds which contain cyclic moieties is generally carried out according to the process of Cava et al, J.Am.Soc., 20, 6022 (1955). Illustrative 4-oxoheptanedioic acid compounds or their ester or acid halide derivatives include:
4-oxoheptanedioic acid,
dimethyl 4-oxoheptanedioate,
2,6-dimethyl-4-oxoheptanedioic acid,
2,3,5,6-tetramethyl-4-oxoheptanedioylchloride,
3,5-diethyl-4-oxoheptanedioic acid,
di-n-propyl 2,6-di-n-butyl-4-oxoheptanediate,
1-carbomethoxy-3,3,5,5-tetramethyl-4-oxo-heptanoic acid,
di(2-carboxyphenyl)ketone,
di(2-carbethoxyphenyl)ketone,
di(2-chlorocarbonylphenyl)ketone,
3-(2-carboxybenzoyl)propionic acid,
ethyl 3-(2-carbethoxybenzoyl)propionate, and
3-(2-carboxy-4-methylbenzoyl)butyrylchloride.

Preferred are those compounds of the formula VI wherein each Q is hydroxy or methoxy, especially hydroxy and wherein each Z independently is C(Z')₂ in which each Z' independently is hydrogen or methyl or wherein Z₁ and Z₂ and/or Z₃ and Z₄ form part of a benzene structure.

The spirodilactones of the formula VII are conveniently produced by the process of Pariza et al., Synthetic Communications, Vol. 13 (3), pp. 243-254 (1983). Production of the spirodilactones having additional rings fused to the spiro ring structure is by the process of U.S. patent No. 1,999,101.

Illustrative 1,6-dioxaspiro[4,4]-nonane-2,7-diones include
1,6-dioxaspiro[4,4]nonane-2,7-dione,
3,8-dimethyl-1,6-dioxaspiro[4,4]nonane-2,7-dione,
3,4,8,9-tetramethyl-1,6-dioxaspiro[4,4]nonane-2,7-dione,
3,3,8,8-tetramethyl-1,6-dioxaspiro[4,4]nonane-2,7-dione,
3,3,4,4,8,8,9,9-octomethyl-1,6-dioxaspiro[4,4]nonane-2,7-dione,
3,4,8,9,tetrafluoro-1,6-dioxaspiro[4,4]nonane-2,7-dione,
3-methyl-8,9-benzo-1,6-dioxaspiro[4,4]nonane-2,7-dione,
8,9-benzo-1,6-dioxaspiro[4,4]nonane-2,7-dione, and
3,4,8,9-dibenzo-1,6-dioxaspiro[4,4]nonane-2,7-dione.

Preferred are those compounds of the formula VII wherein each Z independently is C(Z')₂ in which each Z' independently is hydrogen or methyl or wherein Z₁ and Z₂ and/or Z₃ and Z₄ form part of a benzene ring.

The reaction of the 4-oxoheptanedioic acid or its ester or acid halide derivative of the formula VI and the primary amino compound of the formula V is referred to as route A and the reaction of the 1,6-dioxaspiro[4,4]-nonane-2,7-dione of the formula VII and the primary amino compound of the formula V is referred to as route B.

In the process of the invention the aminophenol is employed in an amount of from 1 mole to 5 moles per mole of reactant VI (i.e. for route A the 4-oxoheptanedioic acid compound or an ester or acid halide derivative thereof, and for route B the spirodilactone) but preferably in an amount of from 1.5 mole to 3 moles per mole of reactant VI.

The reaction of reactant VI and the hydroxy containing primary amino compound of the formula V takes place in a liquid reaction diluent. Suitable reaction diluents are polar diluents in which reactant VI and the hydroxy containing primary amino compound are soluble, at least at reaction temperature, and which are inert to the reactants and the dihydric spirodilactam product. Suitable diluents include dialkylketones such as methyl ethyl ketone, methylisobutylketone and diisopropylketone; esters such as butylacetate and methyl-2-ethylhexanoate; ethers including acyclic ethers such as diethyleneglycol dimethyl ether and triethylene glycol diethyl ether as well as cyclic ethers such as tetrahydrofuran and dioxane; N,N-dialkylamides such as N,N-dimethylacetamide, N,N-dimethylformamide and N,N-diethylacetamide and sulphur containing diluents such as dimethylsulphoxide and sulfolane. Of these diluent types the N,N-dialkylamides are a preferred class, particularly N,N-dimethylacetamide.

Reactant VI and the hydroxy containing primary amino compound are contacted under reaction conditions in solution in the reaction diluent by conventional methods such as shaking, stirring, or refluxing. Suitable reaction temperatures are from 80 °C to 250 °C, preferably of from 100 °C to 200 °C, depending in part on the particular diluent employed and the reaction pressure. Suitable reaction pressures are sufficient to maintain the reaction mixture in a liquid phase and vary of from 1 atmosphere to 20 atmospheres, preferably of from 1 atmosphere to 5 atmospheres. Subsequent to reaction the product mixture is separated and the dihydric spirodilactam product is recovered by conventional methods such as precipitation, selective extraction or destillation.

The conversion of the dihydric spirodilactam to the glycidyloxy substituted derivative according to the present invention is conducted in the liquid phase at relatively elevated temperature. The epihalohydrin reactant should be employed in a quantity of at least 2 moles per mole of the dihydric spirodilactam because of the 2:1 stoichiometry of the reaction and preferably in a quantity of at least 4 moles per mol of dihydric spirodilactam. Frequently a substantial excess of epihalohydrin is employed to serve as a reaction diluent as well as a reactant. Alternatively other reaction diluents such as aromatic hydrocarbons including toluene and xylene can be utilized so long as they are relatively inert to the reactants and the epoxy product. The reaction typically is conducted at a temperature of from 80 °C to 180 °C and a reaction pressure of from 1 atmosphere to 5 atmospheres.

Better results are obtained when the reaction of epihalohydrin and dihydric spirodilactam is conducted in the presence of a quaternary phosphonium salt as catalyst, preferably an alkyltriphenylphosphonium halide. Ethyltriphenylphosphonium iodide or bromide comprise preferred catalysts for the process. The initial reaction product of the epihalohydrin and dihydric spirodilactam is, without isolation, treated with a strong base, typically aqueous sodium hydroxide while the water both present and formed is removed by distillation under conditions of approximately the normal boiling temperature of the mixture. Such a two step conversion is entirely conventional for the conversion of hydroxyaryl containing cyclic compounds to the corresponding glycidyloxyarylderivative. For example, 2,2-di(4-hydroxyphenyl)propane is converted commercially to the corresponding diglycidyl ether by this technique. Further illustrations of the process are found for example in the Encyclopaedia of Polymer Science and Technology, 1968, Vol. 6, 209-222.

Acrylyl- or methacrylyl derivatives of dihydric spirodilactams according to the present invention are typically produced by reacting the alkali metal salt, preferably the sodium or potassium salt, of the dihydric spirodilactam with an acrylyl- or methacrylyl alkoxide or with an acrylyl- or methacrylylhalide, in the presence of a diluent.

Allyl- or propargyloxy derivatives of dihydric spirodilactams according to the present invention are typically produced by reacting the alkali metal salt, preferably the sodium or potassium salt, of the dihydric spirodilactam with an allyl- or propargylhalide, preferably bromide or chloride, in the presence of a reaction diluent.

The alkali metal salt of the dihydric spirodilactam can be produced by contacting the dihydric spirodilactam with stoichiometric quantity of an alkali metal hydroxide per mole of dihydric spirodilactam. The reaction is conducted in the liquid phase in a suitable reaction solvent such as N,N-dimethylacetamide or N,N-dimethylformamide while removing the water present or formed by distillation, preferably azeotropic distillation employing a second solvent such as toluene or ethylbenzene with which water forms an azeotrope.

The alkali metal salt of the spirodilactam is isolated if desired by conventional methods such as solvent removal but the salt is typically used in situ, in the media of its production, for the reaction with the unsaturated moiety compound.

For convenience the terms "unsaturated moiety compound" are used throughout the specification to indicate a compound selected from the group of compounds consisting of acrylyl- and methacrylyl alkoxides and acrylyl-, methacrylyl-, allyl- and propargylhalides.

The reaction of the alkali metal salt of the dihydric spirodilactam and the unsaturated moiety compound is preferably carried out in polar diluents in which the compounds undergoing reaction are soluble at least under reaction conditions. Suitable reaction diluents include N-alkylamides, such as N,N-dimethylacetamide, N,N-dimethylformamide and N-methyl-2-pyrrolidone, phenols such as phenol and m-cresol and sulphur containing solvents such as sulfolane and dimethylsulphoxide.

The unsaturated moiety compound should be employed in a molar amount at least equal to or in excess over the alkali metal salt. Molar ratios of reactants from 5:1 to 1:1 are preferred. Ratios higher than 2 are most preferred because of the 2:1 stoichiometry.

The reaction is effected by charging the unsaturated moiety compound, the alkali metal salt of the dihydric spirodilactam and the reaction diluent to a suitable reactor and maintaining the reaction mixture under reaction conditions. Alternatively, however, first the alkali metal salt is produced in situ by providing the dihydric spirodilactam to the reaction mixture and adding a sufficient amount of an alkali metal hydroxide, carbonate or bicarbonate to neutralize the spirodilactam whereafter the unsaturated moiety compound is added. In this modification it is useful to add a second solvent with which the water present or formed during neutralization is removed as an azeotrope. Toluene and ethylbenzene are illustrative of suitable azeotropic distillation solvents.

The reaction to produce the unsaturated ether derivatives of the dihydric spirodilactams is suitably conducted at a temperature of from -30 °C to 200 °C preferably of from -10 °C to 175 °C and at reaction pressures preferably from 0.8 to 5 atmospheres.

The produced ether derivatives of the dihydric spirodilactams find utility as thermosetting resins which are employed in the production of cured or crosslinked products useful as surface coatings, in adhesive formulations and in fibre-reinforced composites wherein, for example the reinforcing fibre is glass or carbon. Such products are produced by conventional methods. The cured products are also useful in the production of hollow objects as by filament winding and are employed as impregnating and casting resins.

The curing of the unsaturated ether derivatives according to the present invention is accomplished by conventional methods such as thermal heating, e.g. to a temperature over 200 °C, or by photochemical excitation, e.g. as by exposure to high energy radiation, by catalyzed polymerization employing cationic or anionic catalysts or by reaction with a polyfunctional curing agent.

Anionic polymerization uses alkali metal alcoholates, hydroxides or amides as catalyst while typical cationic polymerization catalysts are inorganic or organic acids or are lewis acids. Such cationic catalysts include sulphuric acid, phosphoric acid, p-toluenesulfonic acid, borontrifluoride and tin tetrachloride. Cationic and anionic catalysts are generally employed in a quantity of from 0.05% by weight to 5% by weight, based on total composition. In a preferred modification the unsaturated ether derivatives of the present invention are cured by heating with a substantial amount, e.g., from 20% by weight to 50% by weight based on total composition, of the polyfunctional curing agent. Such curing agents have at least two substituents with multiple bonds between adjacent atoms. Preferred polyfunctional curing agents have up to 30 carbon atoms and contain functional groups selected from alkenyl, alkynyl, styrylmethyl, cyanato or maleimido. Particularly preferred are the maleimide-substituted polyfunctional curing agents, especially di(4-maleimidophenyl)methane.

Starting from an aromatic dihydric compound ("dihydroxyaryl") and a di(glycidyloxy)-substituted compound ("diglycidylether") of which at least a part of either of these compounds is a spirodilactam, a 1,6-diazaspiro[4,4]nonane-2,7-dione derivative can be prepared that is a linear polyhydric polyether. Preferably the polydric polyethers are represented by formula I wherein m is larger than 0.

More preferably, p in formula (I) is either 1 or 0, representing an alternating copolymer and a homopolymer respectively. Even more preferable, divalent radical P is a di(oxyphenyl)propane moiety. Still more preferably, P is a 2,2-di(4-oxyphenyl)propane moiety. However, even more preferred 1,6-diazaspiro[4,4]nonane-2,7-dione derivatives are those in which each n is 0.

It will be apparent that the dihydroxyaryl and the diglycidyl ether will combine in an equimolar relationship to give the polymeric product. Although the ratio of diphenol to diglycidylether to be employed is suitably of from 3:1 to 1:3 the reactants are preferably employed in a ratio which is substantially equimolar. Reaction is conducted by mixing the reactant and maintaining the mixture under polymerization conditions at an elevated temperature. Reaction temperatures to be suitably employed are above 150°C but preferably above 180 °C. An atmospheric reaction pressure is useful although superatmospheric pressures may be utilized. Reactant contact during the reaction is preferably maintained by conventional methods such as shaking or stirring. The reaction conditions, particularly the reaction temperature, will control to some extent the molecular weight of the polyhydric 1,6-diazaspiro[4,4]nonane-2,7-dione derivative. Derivatives of molecular weight of from 10,000 to 100,000 are preferred because of the properties they exhibit. It is useful on occasion to employ a polymerization catalyst which is preferably a phosphonium salt particularly a phosphoniumhalide although phosphonium acetates and phosphonium bicarbonates are also useful. Such catalysts are conventional in processes of this type and often are alkyltriphenylphosphonium salts. Ethyltriphenylphosphonium salts particularly ethyltriphenylphosphonium bromide or ethyltriphenylphosphonium iodide are preferred. The phosphonium salt is employed in catalytic quantities. Amounts of the phosphonium salt of up to 5% by mole, based on total reactants, are satisfactory.

Subsequent to reaction the polymeric product is recovered by conventional methods such as precipitation, selective extraction or distillation. The product is purified by conventional techniques such as dissolving the product mixture in a suitable solvent, e.g., an ether such as tetrahydrofuran, and precipitating the polymer with an alcohol such as methanol.

The polyhydric polyethers of the invention are characterized by high glass transition temperatures typically above 150 °C or higher. They find utility in the applications conventionally associated with phenoxy resins but additionally are useful for engineering applications such as moulded containers for food and drink which are frequently exposed to elevated temperatures. The polymers can be processed by means of the usual techniques such as injection, compression or blow moulding to prepare films and shaped articles. In general, the novel spirodilactam products and the polymers prepared thereof, may be cured using conventional curing agents typically in the art of thermosetting resins. The invention is further illustrated by the following examples which should not be construed as limiting.

### Example I

A mixture of 100 g (0.574 mole) of 4-oxoheptanedioic acid, 260.7 g (1.148 mole) of 2-(4-aminophenyl)-2-(4-hydroxyphenyl)propane and 250 ml of N-methyl-2-pyrrolidone was placed in a 3 litre round-bottomed flask equipped with a mechanical stirrer and a condenser. While being stirred, the mixture was warmed to 160°C and maintained at that temperature for 72 hours. After cooling, the N-methyl-2-pyrrolidone was removed under reduced pressure and methanol was added to precipitate the product. The product was washed several times with methanol and then dried in a vacuum oven at 80 °C-90 °C for 24 hours. A nuclear magnetic resonance analysis of the product indicated a major amount of a compound of the structure 1,6-di[4-(4-hydroxyphenylisopropyl)phenyl]-1,6-diazaspiro[4,4]nonane-2,7-dione.

### Example II

The process according to example 1 was repeated except that 90 g of 1,6-dioxaspiro[4,4]nonane-2,7-dione was employed instead of the 4-oxoheptanedioic acid. Nuclear magnetic resonance analysis indicated the formation of the same reaction product as found in Example I.

### Example III

A mixture of 13.6 g (0.05 mole) of di(2-carboxyphenyl)ketone, 10.99 g (0.1 mole) of 4-aminophenol and 50 ml of N-methyl-2-pyrrolidone was placed in a 500 ml round-bottomed flask equipped with a mechanical stirrer and a condenser. While being stirred, the mixture was warmed to 190 °C and maintained at that temperature for 7 days. After cooling the N-methyl-2-pyrrolidone was removed under reduced pressure and methanol was added to precipitate the product. The precipitated product was washed several times with methanol and dried in a vacuum oven at 150 °C for 24 hours. The product had a melting point above 350 °C and nuclear magnetic resonance spectra of the product was consistent with the structure 1,6-di(4-hydroxyphenyl)-3,4,8,9-dibenzo-1,6-diazaspiro[4,4]nonane-2,7-dione.

### Example IV

A mixture of 0.03 mole of 1,6-di(4-hydroxyphenyl-3,4,8,9-dibenzo-1,6-diazaspiro[4,4]nonane-2,7-dione, 0.05 g of ethyltriphenylphosphoniumbromide and 150 ml of epichlorohydrin was placed in a 500 ml round-bottomed flask equipped with a mechanical stirrer and a condenser. The mixture was stirred while warmed to 120 °C and maintained at 110 °C-120 °C for 4 hours. The mixture was then cooled to 80 °C-90 °C while stirring continued and 5.0 g of 50 % aqueous sodium hydroxide was added dropwise as the water present or formed was removed by distillation. After addition of the sodium hydroxide, the unreacted epichlorohydrin was removed by distillation under reduced pressure and methanol was added to precipitate the product. The precipitated product was washed several times with methanol and then dried in a vacuum oven for 24 hours. The nuclear magnetic resonance spectra were consistent with the structure 1,6-di(4-glycidyloxyphenyl)-3,4,8,9-dibenzo-1,6-diazaspiro[4,4]nonane-2,7-dione.

### Example V

The process according to example IV was repeated except that 0.03 mole of 1,6-di[4-(4-hydroxyphenylisopropyl)phenyl]-1,6-diazaspiro[4,4]nonane-2,7-dione was employed instead of 1,6-di(4-hydroxyphenyl)-3,4,8,9-dibenzo-1,6-diazaspiro[4,4]nonane-2,7-dione Nuclear magnetic resonance analysis indicated the formation of a reaction product of the structure 1,6-di[4-(4-glycidyloxyphenylisopropyl)phenyl]-1,6-diazaspiro[4,4]nonane-2,7-dione.

### Example VI

To a three litre three-necked flask was added a mixture of 202.8 g (0.6 mole) of 1,6-di(4-hydroxyphenyl)-1,6-diazaspiro[4,4]nonane-2,7-dione, 91.22 g (0.6 mole) of potassium carbonate, 200 ml of toluene and 1 litre of N,N-dimethylacetamide. The mixture was heated to 150 °C-160 °C and water was removed by azeotropic distillation. When the water removal was complete, the temperature was lowered to 80 °C-90 °C and 200.2 g (1.66 mole) of allylbromide in 200 ml of N,N-dimethylacetamide was added over the next 80 minutes. The reaction temperature was then raised for 12 hours. The resulting mixture was cooled and filtered and the concentrated solution was then poured slowly into a mixture of hexane and ether. The precipitated product was recovered by filtration and dried in a vacuum oven at 80 ^{o}C. The product had a melting point of 152 ^{o}C-155 ^{o}C and the nuclear magnetic resonance spectra of the product were consistent with the structure 1,6-di(4-allyloxyphenyl)-1,6-diazaspiro[4,4]nonane-2,7-dione.

This product was mixed with an equal portion by weight of bismaleimide, i.e. di(4-maleimidophenyl)methane.
The resulting mixture was heated at 170 °C for 2 hours, at 210 °C for 2 hours and finally at 250 °C for 6 hours. The resulting cured product was insoluble in common solvents and had a glass transition temperature of 312 °C.

### Example VII

To a three litre three-necked flask was added a mixture of 135.2 g (0.4 mole) of 1,6-di(4-hydroxyphenyl)-1,6-diazaspiro[4,4]nonane-2,7-dione, 58.0 g (0.42 mole) of potassium carbonate, 500 ml of N,N-dimethylformamide and 200 ml of toluene. The mixture was heated to 150 °C-160 °C and water was removed by azeotropic distillation. When the water removal was complete, the temperature was lowered to 80 °C-90 °C and 95.2 g (0.8 mole) of propargylbromide in 100 ml of dimethylformamide was added over a 2.5 hour period. The reaction temperature was raised to 100 °C and maintained at that temperature for 12 hours. The resulting solution was cooled, filtered and reduced in volume upon a rotary evaporator. The concentrated solution was then poured slowly into water to give a precipitated product which was recovered by filtration and dried in a vacuum oven at 80 °C. The product had a melting point of 210 ^{o}C-216 °C and the nuclear magnetic resonance spectra of the product were consistent with the structure 1,6-di(4-propargyloxyphenyl)-1,6-diazaspiro[4,4]nonane-2,7-dione.

This product was cured by heating for 12 hours at 210 °C. The cured product had a glass transition temperature of 305 °C.

## Claims

1. A 1,6-diazaspiro[4,4]nonane-2,7-dione derivative having the formula I: 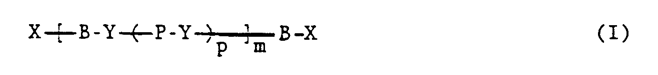 in which B has the formula II: 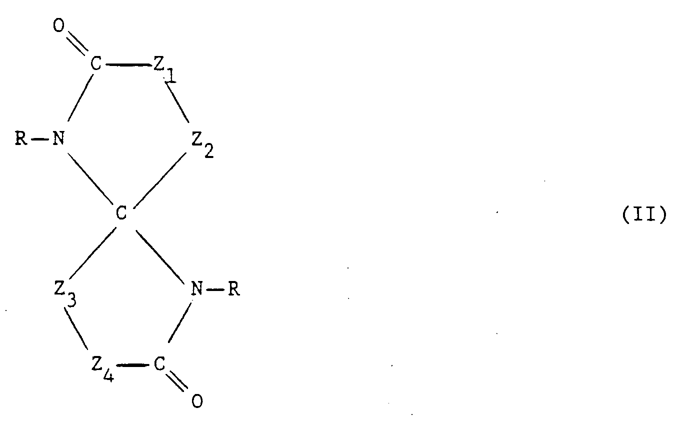 wherein
a) Z₁, Z₂, Z₃, Z₄ are independently
- C(Z')₂ in which Z' independently is hydrogen or C₁₋₄ alkyl, or
- such that two adjacent Z atoms form part of a benzene ring;
b) each R is independently a bivalent group of the formula III or IV: 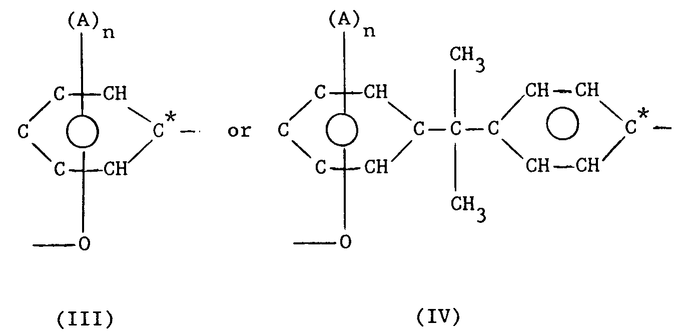 wherein A is a substituent replacing hydrogen selected independently from a C₁₋₄ alkyl or halogen atom, n independently is 0, 1 or 2 and the free valence bond of C* is linked to a ring nitrogen atom of the spirodilactam of the formula II;
c) m ≧ 0;
d) each X independently is hydrogen or a glycidyl-, acrylyl-, methacrylyl-, allyl- or propargyl group, with the proviso that when X is acrylyl, methacrylyl, allyl or propargyl then m is 0;
e) Y is 2-hydroxy-1,3-propadiyl;
f) each P independently is a di(oxyphenyl)propane moiety;
g) p is on average of from 0 to 3;
with the proviso that when R is a bivalent group of the formula III and simultaneously Z₂ and Z₃ are each CH₂ and simultaneously Z₁ and Z₄ are C(Z')₂ wherein each Z' independently represents hydrogen or a C₁₋₄ alkyl, then each X is not hydrogen or glycidyl.

2. A 1,6-diazaspiro[4,4]nonane-2,7-dione derivative as claimed in claim 1, wherein each Z independently is C(Z')₂ in which each Z' independently is hydrogen or methyl.

3. A 1,6-diazaspiro[4,4]nonane-2,7-dione derivative as claimed in claim 1, wherein Z₁ and Z₂ and/or Z₃ and Z₄ form part of a benzene structure fused to the spiro ring system.

4. A 1,6-diazaspiro[4,4]nonane-2,7-dione derivative as claimed in claims 1, 2 or 3, wherein n is 0.

5. A 1,6-diazaspiro[4,4]nonane-2,7-dione derivative as claimed in any of claims 1 to 4, wherein m is 0 and each X is either hydrogen or a glycidyl-, allyl- or propargyl group.

6. A 1,6-diazaspiro[4,4]nonane-2,7-dione derivative as claimed in any one of claims 1 to 4, wherein m is > 0 and p is 0 or 1.

7. A 1,6-diazaspiro[4,4]nonane-2,7-dione derivative as claimed in claim 6, wherein m is selected such that the derivative has a molecular weight of from 10,000 to 100,000.

8. A 1,6-diazaspiro[4,4]nonane-2,7-dione derivative as claimed in claim 6 or 7, wherein the divalent radical P is derived from 2,2-di(4-hydroxyphenyl)propane.

9. A cured composition comprising at least one 1,6-diazaspiro[4,4]nonane-2,7-dione derivative as claimed in any one of the claims 1 to 8.

## Patentansprüche

1. Ein 1,6-Diazaspiro[4,4]nonan-2,7-dionderivate mit der nachstehenden Formel I 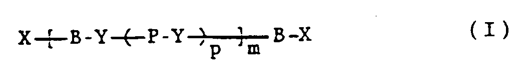 in welcher B die Formel II hat 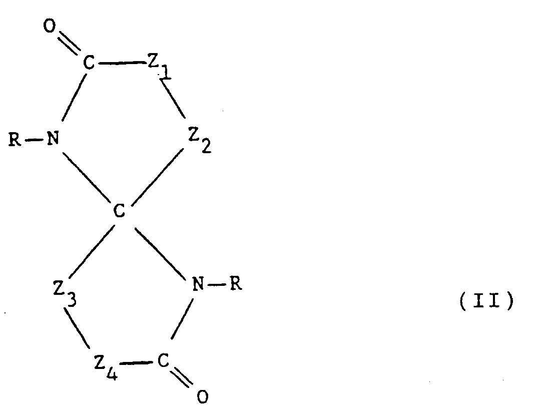 in welcher
a) Z₁, Z₂, Z₃, Z₄ unabhängig voneinander die folgende Bedeutung haben
- C(Z')₂, wobei Z' unabhängig voneinander Wasserstoff oder C₁₋₄-Alkyl bedeutet, oder
- zwei benachbarte Z-Atome Teil eines Benzolringes bilden;
b) jedes R unabhängig voneinander eine zweiwertige Gruppe der Formel III oder IV darstellt 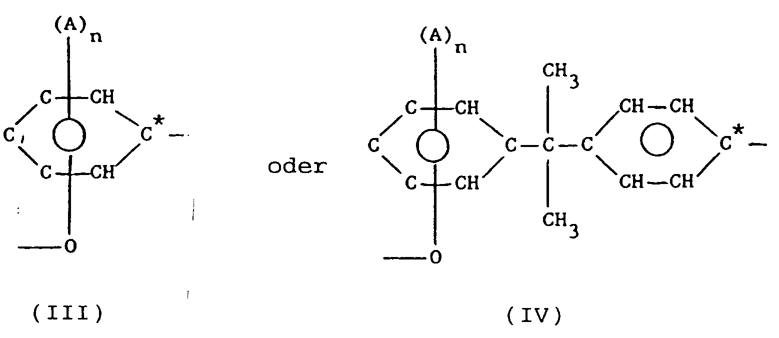 in welchen A ein Wasserstoff ersetzender Substituent ist, unabhängig ausgewählt aus einer C₁₋₄-Alkylgruppe oder einem Halogenatom, n unabhängig voneinander den Wert 0, 1 oder 2 hat, und die freie Bindung des C*-Atoms an ein Ringstickstoffatom des Spirodilactams der Formel II gebunden ist;
c) m ≧ 0 ;
d) jedes X unabhängig voneinander Wasserstoff oder eine Glycidyl-, Acrylyl-, Methacrylyl-, Allyl- oder Propargylgruppe ist, mit der Maßgabe, daß wenn X die Bedeutung Acrylyl, Methacrylyl, Allyl oder Propargyl hat, dann m den Wert 0 hat;
e) Y die 2-Hydroxy-1,3-propadiylgruppe ist;
f) jedes P unabhängig voneinander ein Di(oxyphenyl)propan-Molekülteil ist;
g) p im Durchschnitt einen Wert im Bereich von 0 bis 3 hat;
mit der Maßgabe, daß wenn R eine zweiwertige Gruppe der Formel III bedeutet und Z₂ und Z₃ gleichzeitig jeweils eine Gruppe CH₂ darstellen und Z₁ und Z₄ die Bedeutung C(Z')₂ haben, wobei jedes Z' unabhängig Wasserstoff oder eine C₁₋₄-Alkylgruppe ist, jedes X weder Wasserstoff noch Glycidyl ist.

2. Ein 1,6-Diazaspiro[4,4]nonan-2,7-dionderivat wie in Anspruch 1 beansprucht, in welchem jedes Z unabhängig die Bedeutung C(Z')₂ hat, wobei jedes Z' unabhängig Wasserstoff oder Methyl ist.

3. Ein 1,6-Diazaspiro[4,4]nonan-2,7-dionderivat wie in Anspruch 1 besprucht, in welchem Z₁ und Z₂ und/oder Z₃ und Z₄ Teil einer Benzolstruktur bilden, welche mit dem Spiroringsystem verschmolzen ist.

4. Ein 1,6-Diazaspiro[4,4]nonan-2,7-dionderivat wie in Anspruch 1, 2 oder 3 beansprucht, in welchem n den Wert 0 hat.

5. Ein 1,6-Diazaspiro[4,4]nonan-2,7-dionderivat wie in irgendeinem der Ansprüche 1 bis 4 beansprucht, in welchem n den Wert 0 hat und jedes X entweder Wasserstoff oder eine Glycidyl-, Allyl- oder Propargylgruppe ist.

6. Ein 1,6-Diazaspiro[4,4]nonan-2,7-dionderivat wie in irgendeinem der Ansprüche 1 bis 4 beansprucht, in welchem m > 0 ist und p den Wert 0 oder 1 hat.

7. Ein 1,6-Diazaspiro[4,4]nonan-2,7-dionderivat wie in Anspruch 6 beansprucht, in welchem m derart ausgewählt ist, daß das Derivat ein Molekulargewicht im Bereich von 10000 bis 100000 hat.

8. Ein 1,6-Diazaspiro[4,4]nonan-2,7-dionderivat wie in Anspruch 6 oder 7 beansprucht, in welchem das zweiwertige Radikal P sich von 2,2-Di-(4-hydroxyphenyl)propan ableitet.

9. Eine gehärtete Zusammensetzung welche mindestens ein 1,6-Diazaspiro[4,4]nonan-2,7-dionderivat, wie in irgendeinem der Ansprüche 1 bis 8 beansprucht, enthält.

## Revendications

1. Un 1,6-diazaspiro[4,4]nonane-2,7-dione ayant la formule I : 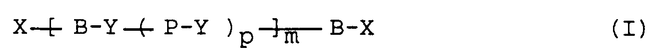 dans laquelle B possède la formule II : 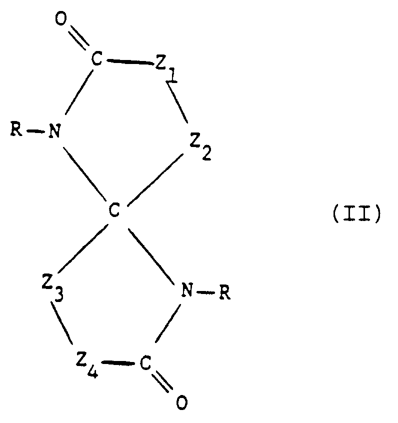 dans laquelle
a) Z₁, Z-₂, Z-₃, Z₄ représentent indépendamment
- C(Z')₂ où Z' représente indépendamment de l'hydrogène ou un alkyle en C₁ à C₄, ou bien sont tels que les deux atomes Z adjacents font partie d'un noyau benzénique ;
b) chaque R représente indépendamment un groupe bivalent de la formule III ou IV : 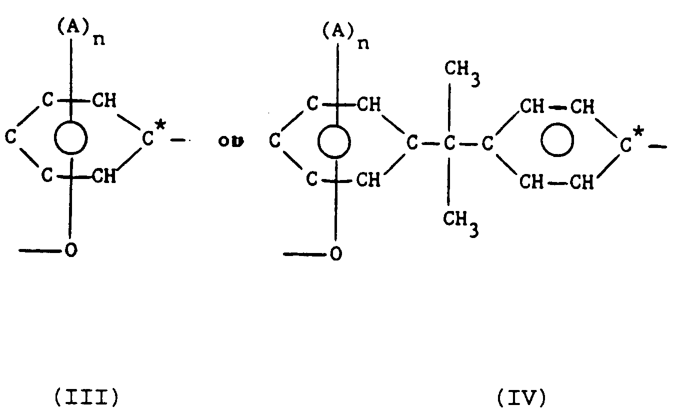 dans lequel A est un substituant remplaçant l'hydrogène choisi indépendamment parmi les alkyles en C₁ à C₄ ou les atomes d'halogène, n est indépendamment 0, 1 ou 2 et la liaison valence libre de C* est liée à un atome d'hydrogène du cycle du spirodilactame de formule II ;
c) m est supérieur ou égal à 0 ;
d) chaque X représente indépendamment de l'hydrogène ou un groupe glycidyle, acrylyle, méthacrylyle, allyle ou propargyle, sous la condition que, lorsque x est un groupe acrylyle, méthacrylyle, allyle ou propargyle, alors m est 0 ;
e) Y est un radical 2-hydroxy-1,3-propadiyle ;
f) chaque P représente indépendamment une partie di(oxyphényl)propane ;
g) p est en moyenne de 0 à 3 ;
sous la condition que lorsque R est un groupe bivalent de la formule III et que Z₂ et Z₃, simultanément, représentent chacun CH₂ et simultanément Z₁ et Z₄ sont C(Z')₂, où chaque Z' représente indépendamment de l'hydrogène ou un alkyle en C₁ à C₄, alors chaque X n'est ni de l'hydrogène ni du glycidyle.

2. Un dérivé de la 1,6-diazaspiro[4,4]nonane-2,7-dione, tel que revendiqué dans la revendication 1, dans lequel chaque Z est indépendamment C(Z')₂ où chaque Z' est indépendamment de l'hydrogène ou du méthyle.

3. Un dérivé de 1,6-diazaspiro[4,4]nonane-2,7-dione, tel que revendiqué dans la revendication 1, dans lequel Z₁ et Z₂ et/ou Z₃ et Z₄ forment une partie d'une structure benzénique fusionnée à un système cyclique spiro.

4. Un dérivé de 1,6-diazaspiro[4,4]nonane-2,7-dione, tel que revendiqué dans les revendications 1, 2 ou 3, dans lequel n est 0.

5. Un dérivé de 1,6-diazaspiro[4,4]nonane-2,7-dione tel que revendiqué dans l'une quelconque des revendications 1 à 4, dans lequel m est 0 et chaque X est soit de l'hydrogène soit un groupe glycidylique, allylique ou propargylique.

6. Un dérivé de 1,6-diazaspiro[4,4]nonane-2,7-dione tel que revendiqué dans l'une quelconque des revendications 1 à 4, dans lequel m est supérieur à 0 et p est 0 ou 1.

7. Un dérivé de 1,6-diazaspiro[4,4]nonane-2,7-dione tel que revendiqué dans la revendication 6, dans lequel m est choisi de façon que le dérivé présente un poids moléculaire de 10.000 à 100.000.

8. Un dérivé de 1,6-diazaspiro[4,4]nonane-2,7-dione tel que revendiqué dans la revendication 6 ou 7, dans lequel le radical divalent P est dérivé du 2,2-di(4-hydroxyphényl)propane.

9. Une composition durcie comprenant au moins un dérivé de 1,6-diazaspiro[4,4]nonane-2,7-dione telle que revendiquée dans l'une quelconque des revendications 1 à 8.
